(19) Europäisches Patentamt
European Patent Office
Office européen des brevets

(11) Numéro de publication: **0 131 176**
**B1**

# (12) FASCICULE DE BREVET EUROPÉEN

(45) Date de publication du fascicule du brevet: **16.12.87**

(51) Int. Cl.⁴: **C 09 K 15/34,** A 61 K 7/00, A 23 L 3/34

(21) Numéro de dépôt: **84106936.2**

(22) Date de dépôt: **16.06.84**

(54) **Protection antioxygène des produits alimentaires et cosmétiques.**

(30) Priorité: **06.07.83 CH 3707/83**

(43) Date de publication de la demande: **16.01.85 Bulletin 85/3**

(45) Mention de la délivrance du brevet: **16.12.87 Bulletin 87/51**

(84) Etats contractants désignés: **AT BE DE FR GB IT NL SE**

(73) Titulaire: **SOCIETE DES PRODUITS NESTLE S.A., Case postale 353, CH-1800 Vevey (CH)**

(72) Inventeur: **Farr, David Robert, Les Fleurettes, CH-1817 Brent (CH)**
Inventeur: **Magnolato, Danière, Avenue de la Cressire 5a, CH-1814 La Tour de Peilz (CH)**
Inventeur: **Loeliger, Jürg, Chemin de Pierre à Fleur 6, CH-1802 Corseaux (CH)**

(56) Documents cité:
**EP-A-0 009 661**

**CHEMICAL ABSTRACTS, vol. 98, no. 5, 31 janvier 1983, page 539, no. 33086n, Columbus, Ohio, USA; H. YOSHINAGA: "Application of antioxidants containing gallic acid to foods"**
**CHEMICAL ABSTRACTS, vol. 83, no. 23, 8 décembre 1975, page 323, no. 191631c, Columbus, Ohio, USA;**
**CHEMICAL ABSTRACTS, vol. 97, no. 23, 6 décembre 1982, page 459, no. 196887q, Columbus, Ohio, USA; H. YOSHINAGA et al.: "Use of gallic acid as antioxidant for food"**
**CHEMICAL ABSTRACTS, vol. 69, no. 23, 2 décembre 1968, page 8900, no. 95240q, Columbus, Ohio, USA; M.A. JOSYLN et al.: "Leucoanthocyanins and related phenolics of carob pods (Ceratonia siliqua)"**

(56) Documents cité: (suite)
**RIECHSTOFFE, AROMEN, KÖRPERPFLEGEMITTEL, vol. 20, no. 3, mars 1970, pages 94,97,98, Gerhard R. Barsch-Fachverlag, Hannover, DE; G. LINDEMANN: "Johannisbrot, Fructus Ceratoniae, als Rohstoff in der Kosmetik- und Likör-Industrie"**
**CHEMICAL ABSTRACTS, vol. 86, no. 13, 28 mars 1977, page 249, no. 86078s, Columbus, Ohio, USA; L. ANCARANI ROSSIELLO: "The "Ceratonia siliqua": production, uses, trade"**
**CHEMICAL ABSTRACTS, vol. 98, no. 1, janvier 1983, page 333, no. 3673f, Columbus, Ohio, USA; N. YAMAGUCHI et al.: "Studies on natural antioxidants. V. Antioxidative activity of pigment(s) extracted from cocoa bean husk"**
**FOOD TECHNOLOGY, vol. 37, no. 3, mars 1983, page 122, colonne du milieu, alinéa 2, Chicago, Illinois, USA;**

EP 0 131 176 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

**Description**

La présente invention a trait à la protection des produits alimentaires et cosmétiques contre l'action de l'oxygène.

Les substances antioxygènes à usage alimentaire ou cosmétique doivent avoir une bonne activité antioxygène, être stables, incolores, neutres organoleptiquement et leur inocuité doit être garantie.

On connaît des substances antioxygènes obtenues par voie de synthèse dont l'usage est très répandu, généralement des substances phénoliques, par exemple le butylhydroxyanisol (B.H.A.), le butylhydroxytoluène (B.H.T.) seuls ou en mélange synergique. On connaît l'activité antioxygène d'autres dérivés phénoliques, par exemple le pyrogallol qui présente l'inconvénient d'être relativement toxique ou par exemple les gallates, en particulier les propyl-, octyl- ou lauryl- gallates. Bien que ces substances aient une activité antioxygène marquée, une bonne stabilité, qu'elles soient incolores et que leur inocuité et leur neutralité vis-a-vis des aliments soient reconnues, leur usage est maintenant fortement contesté par nombre de législations alimentaires.

On a cherché a tirer parti du fait que certaines substances antioxygènes sont contenues dans les produits naturels. Par exemple un bon nombre de corps gras sont naturellement protégés contre l'oxydation par les tocophérols qu'ils contiennent mais l'activité de ceux-ci est faible. On a également proposé d'extraire les substances antioxygènes contenues dans les matières végétales, par exemple les épices, en particulier les labiacées comme la sauge ou le romarin. Les méthodes proposées utilisent un agent chimique et sont donc contestables au même titre que les antioxygènes synthétiques ou comprennent l'extraction par solvants des matières végétales broyées suivie en général de traitements poussés de distillation, décoloration et désodorisation des extraits. Si l'activité antioxygène de ces extraits est reconnue, leur application est restreinte car il subsiste pratiquement toujours une coloration, une odeur et un goût résiduels de l'épice mise en oeuvre. De plus, le principe antioxydant découlant, par exemple de BP-A-0009661 est constitué de tannins condensés.

Enfin, on a proposé par exemple dans le brevet japonais No. 71.039.058 de préserver les produits de la mer de l'oxydation par addition de catéchines, gallocatéchines et dérivés 3-galloyl de ces composés, qui sont des tannins naturels condensés composés essentiellement d'esters d'acide gallique et de flavan-3-ols existant dans les feuilles de thé. Selon ce brevet, on extrait les composés antioxygènes par l'éthanol, on décolore l'extrait sur charbon activé et on évapore le solvant.

Le document C.A., vol. 69, No.23, 952409 a trait à des leucoanthocyanines des gousses de caroube et mentionne l' existence de tannins hydrolysables du type galloylglucose dans les gousses de caroube. Il n'y a aucune indication d'une éventuelle activité antioxydante de tels composés.

La présente invention est basée sur la constatation surprenante que certains tannins hydrolysables naturels extraits de végétaux largement disponibles possèdent un pouvoir antioxygène nettement supérieur à celui des tannins condensés du thé tout en étant remarquablement neutres du point de vue de la couleur, de l'odeur et du goût.

L'invention concerne donc un procédé de préservation des produits alimentaires et cosmétiques contre l'action de l'oxygène, caractérisé par le fait qu'on incorpore dans le produit à préserver une quantité efficace de gallotannins extraits à partir d'une matière végétale.

Les gallotannins, très répandus dans le règne végétal, proviennent des racines, gousses, pulpes de fruits, feuilles ou parasites des plantes tels les galles du bois et de l'écorce des arbres.

Les gallotannins utilisables selon l'invention répondent à la formule:

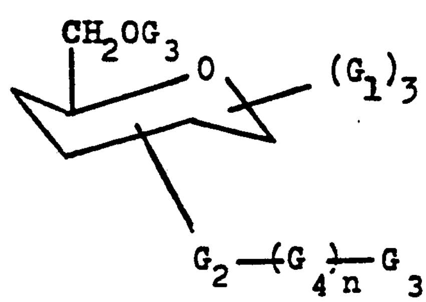

dans laquelle $G_1$ est un résidu d'acide gallique de formule

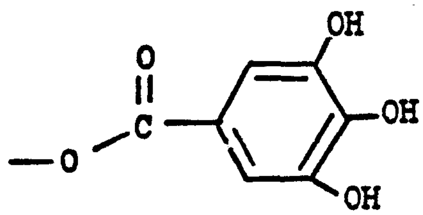

$G_2$ est un résidu d'acide gallique de formule

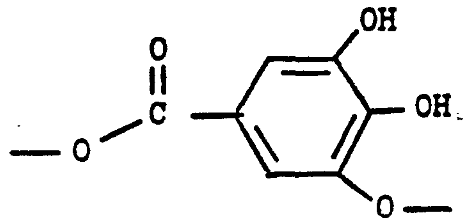

$G_3$ signifie

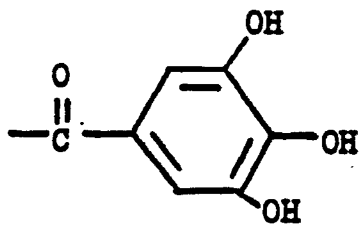

$G_4$ signifie

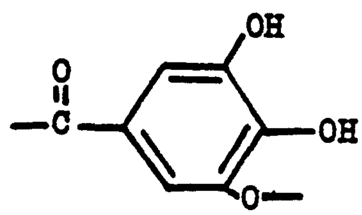

et n est 0,1 ou 2, le groupe -$G_2$-[$G_4$]-$G_3$ étant en position 2, 3 ou 4, les groupes $G_1$ occupant les positions libres du noyau de glucose.

Ces gallotannins sont hydrolysables, ce qui signifie qu'ils sont dégradés facilement en leurs fragments constitutifs de poids moléculaire plus faible par l'action des enzymes, de l'eau chaude, des acides et des bases. Contrairement aux gallocatéchines du thé, le polyol n'est pas condensé ou polycyclique, mais est le glucose.

Une source avantageuse de gallotannins est constituée par les gousses de caroube verte, Ceratonia siliqua, qu'on trouve en abondance et qu'on peut mettre en oeuvre brute ou sous forme de résidu désucré provenant de l'extraction à l'eau de la farine de caroube.

Selon une première forme d'exécution, on extrait la matière végétale par un solvant miscible à l'eau tel que par exemple l'éthanol, le méthanol ou l'acétone, l'acool éthylique étant préféré, car il est alimentaire. On utilisera avantageusement un mélange d'alcool éthylique et d'eau à 90-96 % d'alcool. Les proportions pondérales solvant/matière végétale sont de 100/1 jusqu'à 5/1 et de préférence 20/1. On extrait la matière végétale moulue à une température allant de 20° C jusqu'à la température d'ébullition du solvant utilisé pendant 5 à 30 min. On évapore ensuite le solvant sous vide à une température de 20 à 50° C selon le solvant utilisé et le vide appliqué. On obtient ainsi un extrait brut directement utilisable dans les produits a protéger.

Selon une seconde forme d'exécution, on purifie l'extrait brut séché en le reprenant par un mélange de solvant organique non miscible à l'eau et d'eau, par exemple d'acétate d'éthyle et d'eau dans des proportions en volume d'environ 1/1 à pH voisin de la neutralité, par exemple 6,8-7, de préférence à la température ambiante. On utilise environ 20 parties de liquide d'extraction pour 1 partie en poids d'extrait brut. Ceci permet de séparer les gallotannins des composés solubles dans l'eau, en particulier de l'acide gallique. On sépare la phase organique et on évapore le solvant sous pression réduite à une température de 20 à 50° C selon le solvant utilisé et le vide appliqué. Cet extrait purifié contient essentiellement l'acide tannique et peut être incorporé tel quel dans les produits à protéger.

Selon une troisième forme d'exécution qui est préférée, on procède à une hydrolyse de l'extrait purifié ci-dessus dans un milieu solvant miscible à l'eau, par exemple dans le méthanol après avoir réglé le pH à 6-8 et de

préférence 7-8 (le pH étant mesuré dans le méthanol), par exemple au moyen d'un milieu tampon acétate d'éthyle-acide acétique. Le rapport méthanol/milieu aqueux utilisé est d'environ 10/1 en volume et le rapport extrait purifié/milieu d'extraction d'environ 1/20 en poids. L'extraction a lieu en atmosphère exempte d'oxygène, par exemple sous azote après avoir pris soin de désoxygéner le milieu d'extraction, par exemple par désaération à l'ébullition ou par barbotage d'azote. La température de l'opération est de 20-40°C, de préférence la température ambiante et sa durée 4 à 7 jours.

L'hydrolyse de l'acide tannique conduit à cliver sélectivement les liaisons depsidiques entre deux groupes galloyles, ce qui libère un mélange de penta-, tetra-, trigalloylglucose, de gallate et de digallate de méthyle, le pentagalloylglucose constituant 28 à 51 % du nombre de ces composés, suivant la nature de la matière première et les conditions, en particulier de pH de l'hydrolyse.

Après évaporation du méthanol sous pression réduite, on reprend le résidu par l'eau en proportion résidu/eau d'environ 1/5 en poids. On procède à la dialyse contre l'eau pure avec des membranes de 1,5 à 2 nm (nanomètre) pendant au moins 24 h et de préférence pendant 48 h à la température ambiante. Cette opération permet d'éliminer les petites molécules par exemple le gallate, le digallate de méthyle et les sels utilisés comme tampon. On extrait ensuite le milieu aqueux par exemple par un volume égal d'acétate d'éthyle, on évapore le solvant sous pression réduite et on sèche par exemple par lyophilisation.

En variante, on peut pousser la séparation des polygalloylglucoses jusqu'à recueillir une fraction contenant essentiellement le pentagalloylglucose par chromatographie sur colonne de cellulose en éluant par exemple avec une solution aqueuse d'acide acétique. On extrait alors le nilieu aqueux par l'acétate d'éthyle, on évapore le solvant sous pression réduite et on sèche par exemple par lyophilisation.

Les extraits antioxygènes peuvent être utilisés pour la protection contre l'oxydation de toutes sortes d'aliments ou de produits cosmétiques.

Comme aliments dans lesquels ils peuvent être incorporés on peut citer les matières grasses telles que les huiles végétales ou les graisses animales; les émulsions du type mayonnaise, pâte à tartiner, sauces de salade, crèmes, bouillons cubes, etc...; les produits humides contenant des matières grasses liées tels que la viande, le poisson, par exemple sous forme d'émulsions; les aliments complexes séchés, par exemple les légumes, en particulier les paillettes de pomme de terre, potages déshydratés, produits à base de céréales par exemple structurés, farines lactées, poudres de lait entier, de soja, etc...

Les produits cosmétiques craignant l'oxydation sont sous forme de dispersions aqueuses (lotions, telles que lotions avant ou après rasage), émulsions fluides (laits corporels, laits à démaquiller), crèmes (crème blanche, crème solaire), pâte (masque) etc... Leur protection contre l'oxydation permet en particulier d'éviter les problèmes olfactifs dus au rancissement.

On peut enfin envisager d'incorporer les extraits séchés dans des matières plastiques ou des complexes d'emballage pour produits alimentaires ou cosmétiques.

Des résultats satisfaisants sont obtenus par une incorporation de 0,01 a 1 %, et de préférence de 0,05 à 0,2 % en poids d'extrait, par tout moyen connu, par exemple en solution ou suspension ou émulsion dans des solvants ou des gaz liquéfiés, c'est-à-dire a l'aide d'un vecteur approprié.

On peut évidemment y ajouter d'autres substances antioxydantes dites "secondaires"; celles-ci, par exemple l'acide ascorbique, tartrique, citrique ou l'éthylène diaminetétraacétate ont un effet chélatant vis-à-vis des substances prooxydantes du milieu. D'autres substances antioxydantes secondaires par exemple l'ascorbate, le métabisulfite ou le pyrophosphate de sodium abaissent le potentiel d'oxydo-réduction dudit milieu.

Les exemples qui suivent illustrent la mise en oeuvre de l'invention. Dans ceux-ci, les pourcentages et quantités sont pondérales sauf indication contraire.

**Exemple 1**

a) On place 130 g de caroubes vertes coupées en dés dans un mixer et on les extrait par 400 ml d'une solution d'éthanol à 96 %, à température ambiante et pendant 10 min. On récupère le résidu solide par filtration et on le réextrait 3 fois dans les mêmes conditions. On regroupe les extraits éthanoliques et on les évapore sous vide à 30°C. On obtient 10,4 g d'extrait éthanolique de caroube verte sous forme d'une poudre de couleur brun-vert pâle (extrait brut).

b) Dans le but d'effectuer une comparaison on procède à l'extraction de thé noir et de céréales (orge, avoine, sorgho) dans les conditions utilisées ci-dessous, par référence à l'orge. On broie 500 g d'orge commercial sous forme de particules de 1 mm (valeur moyenne) et on les extrait par 1200 ml d'une solution d'éthanol à 96 %, au reflux, pendant 60 min. On récupère le résidu solide par filtration et on le réextrait 2 fois dans les mêmes conditions. On regroupe les extraits éthanoliques et on les évapore sous vide à 30°C. On obtient 3,1 g d'extrait brut sous la forme d'une poudre légèrement brune.

**Exemple 2**

a) On extrait 500 g de caroubes vertes fraîches découpés en dés dans un mixer par 1000 ml de méthanol pendant 10 min, à température ambiante. On réextrait le résidu 2 fois avec 1000 ml de méthanol. On concentre l'extrait méthanolique à 200 mi par évaporation sous vide a 40°C. On ajoute 250 ml d'eau et on extrait les gallotannins de la phase aqueuse par l'acétate d'éthyle. On répète l'extraction 6 fois avec 500 ml d'acétate d'éthyle chaque fois. On regroupe les phases organiques et on évapore le solvant sous vide à 30°C. On obtient 21,3 g de gallotannins purifiés.

b) Le même schéma d'extraction appliqué à 80 g de noix de galle fraîches permet de récupérer 37 g de gallotannins purifiés.

**Exemple 3**

On dissout 100 g d'acide tannique commercial (Fluka) dans 500 ml de tampon phosphate 1N à pH 7. On ajuste le pH de la solution à 7 par addition de 400 ml d'une solution 1N de hydrogénophosphate de di-sodium. On obtient les gallotannins purifiés par extraction avec 6 fois 250 ml d'acétate d'éthyle. Après séparation des phases, on évapore la phase organique sous vide à 30°C et on obtient 80 g de gallotannins purifiés.

On dissoud les 80 g de gallotannins purifiés dans 180 ml de tampon acétate 0,5N à pH 6. On y ajoute 1600 ml de méthanol, préalablement désoxygéné par chauffage à ébullition pendant 1 $h_r$ et on ajuste le pH de la solution à 7,5 par addition d'acide acétique 2N. On maintient la solution méthanolique de gallotannins pendant 7 jours sous azote, à température ambiante. On évapore ensuite le solvant sous vide à 30°C.

Le tableau I ci-dessous donne les proportions des constituants de l'hydrolysat (en moles %) obtenus à différents pH :

**Tableau I**

| pH de la solution tampon acétate/MeOH | % penta galloyl glucose | % tetra galloyl glucose | % tri-galloyl glucose | % methyl digallate | % methyl gallate |
|---|---|---|---|---|---|
| 7,5 | 51,2 | 19,7 | 3,8 | 4,3 | 21,0 |
| 6,5 | 35,4 | 14,9 | 2,1 | 3,0 | 15,7 |
| 6,0 | 27,9 | 10,9 | 1,3 | 1,8 | 8,2 |

On dissout le résidu constitué principalement de penta-Ogalloyl-D-glucose et de méthylgallate dans 400 ml d'eau et on place la solution dans un sac de dialyse constitué d'une membrane de porosité de 1,5-2,0 nm (nanomètre) pour être dialysé pendant 48 h à température ambiante contre de l'eau pure. La dialyse permet d'éliminer les petites molécules (méthyle gallate essentiellement ainsi que des traces de méthyle digallate et d'acide gallique). On lyophilise le contenu du sac de dialyse et on obtient 38 g de penta-Ogalloyl-D-glucose ayant une pureté de 90 % environ.

On peut éventuellement purifier le penta-O-galloyl-Dglucose par passage sur une colonne de MN cellulose-300G et élution par une solution aqueuse contenant 6 % d'acide acétique. Le penta-O-galloyl-D-glucose ainsi obtenu a une pureté de 97 %. Une colonne de 3 cm de diamètre et 90 cm de hauteur permet de purifier 3 g de produit.

La structure des polygalloylglucoses a été vérifiée par spectrographie de résonance magnétique nucléaire du proton (RMN) ainsi que par chromatographie liquide haute pression par comparaison avec des produits de référence obtenus par voie synthétique, par exemple le pentagalloylglucose préparé à partir de chlorure de tri-O-benzylgalloyle et de β-glucose en présence de pyridine et de chloroforme conduisant au β-penta-O-(benzylgalloyl)glucose transformé en β-penta-O-galloylglucose par hydrogénation.

**Exemple 4**

On a déterminé le pouvoir antioxygène des différents extraits par comparaison avec des extraits antioxygènes du commerce en appliquant le test d'oxydation accélérée selon Rancimat décrit par J. Frank, J. Geil dans "Food Technology <u>1982</u>, 71", dans la graisse de poule à 100°C. Ce test donne une indication très valable du pouvoir de protection des corps gras contre l'action de l'oxygène. Les extraits bruts de sorgho, d'orge, d'avoine et de thé noir ont été obtenus par extraction à l'éthanol selon l'exemple 1b.

Les résultats du temps d'induction en heures sont indiqués dans le tableau II ci-dessous:

**Tableau II**

| Additif | G.P. | Temps d'induction (heures) Concentration (parties par million) 500 | 1000 | 1500 | 2000 |
|---|---|---|---|---|---|
| Extrait brut selon l'exemple 1a | 3 | 6[1] | 7,3 | 10,5 | n.d. |
| Extrait purifié selon l'exemple 2a | 3,8 | 8,2 | n.d. | n.d. | n.d. |
| Pentagalloylglucose (90 % de pureté) selon l'exemple 3 | 3,3 | 7,5 | 10,5 | n.d. | 15,3 |
| Acide tannique du commerce (Fluka) | 3,8 | 10,2 | n.d. | n.d. | n.d. |
| Extrait alcoolique de romarin (Culinar) | 3,8 | 11,1 | n.d. | n.d. | n.d. |
| Tannins chinois du commerce | 3,8 | 11,5 | 16,8 | n.d. | n.d. |
| Extrait brut de sorgho selon l'exemple 1b | 3,5 | 3,2 | 3,5 | n.d. | 3 |
| Extrait brut d'orge selon l'exemple 1b | 3,5 | 3 | 3,2 | n.d. | 3,4 |
| Extrait brut d'avoine selon l'exempl 1b | 3,5 | 3,5 | 3,6 | n.d. | 3,4 |
| Extrait brut de thé noir selon l'exampls 1b | 3,5 | 3 | 3 | n.d. | 3 |
| Acide ellagique | 3,5 | 3 | 3 | n.d. | 3 |

Légende:
n.d.: non déterminé
G.P.: graisse de poule sans additif (référence)
1: ne prend pas en considération que seulement 54 % de l'extrait éthanolique séché est resolubilisé dans l'éthanol.

On voit que les différents extraits de thé et de céréales n'ont pas du tout de propriétés antioxydantes (le sorgho et le thé contiennent des tannins condensés, polymères de catéchine). L'acide ellagique, un dérivé de l'acide gallique condensé n'est pas du tout antioxydant.

D'autre part, une comparaison des couleurs des extraits de romarin et de caroube a 0,1 % en poids dans l'éthanol montre que la solution de romarin du commerce a une absorbance environ 3,7 fois plus grande que celle de la solution de caroube verte selon l'exemple la dans la plage de 650 à 680 nm (nanomètre).

Enfin, l'extrait de caroube verte selon l'exemple 1a n'a pas d'odeur à 20 cm d'un flacon contenant 5 g; par contre, l'extrait de romarin du commerce, même sous forme de traces, est bien odorant.

**Exemple 5**

On a examiné le pouvoir antioxygène sur une émulsion de graisse de poule dans l'eau à 40°C, selon le test d'oxydation accélérée (peroxydation lipidique catalysée par la hémine) selon D. Sandmeier, G. Ziegleder dans "Fette Seifen Anstrichmittel, 84, 11-14 (1982)", des présents extraits en comparaison avec les gallates de méthyl, de propyle et d'octyle. Ce test est un bon modèle pour l'évaluation de la protection des systèmes émulsionnés et des produits humides contre l'oxydation, par exemple les produits alimentaires et cosmétiques numérés ci-dessus, dans lesquels on a incorporé les antioxidants.

Le tableau III ci-dessous donne les résultats des temps d'induction en minutes:

**Tableau III**

| Additif | G.P. | Temps d'induction (minutes) Concentration (parties par million) 200 | 500 |
|---|---|---|---|
| Extrait brut de caroube selon l'exsmple 1a | 4,5 | n.d. | 6 |
| Extrait purifié de caroube selon l'exemple 2 | 5,3 | n.d. | 9,3 |
| Noix de galle purifiée selon l'exemple 2b | 5,2 | n.d. | 9,6 |
| Gallate de propyle | 5,5 | 9 | n.d. |
| Gallate de méthyle | 4,9 | 7,2 | n.d. |
| Gallate d'octyle | 4,9 | 2,2 | n.d. |

Légende:
G.P.: graisse de poule sans additif (référence)
n.d.: non déterminé.

**Exemple 6**

On moud 5 kg de blé complet en particules de 1mm en moyenne et on les disperse dans 9 litres d'eau désionisée à 20°C dans une cuve munie d'agitateur. On procède ensuite à la gélification de l'amidon dans un échangeur à surface raclée chauffé par la vapeur à 135°C pendant environ 1 minute. On sèche ensuite la suspension sur cylindre tournant à 3,5 tours/minutes muni de couteaux racleurs à la température du produit de 125°C. On racle le film formé qu'on floconne dans un granulateur en particules d'environ 1 mm. On remplit des boîtes d'aluminium avec 40 g de flocons et on les sertit.

On mesure le degré d'oxydation de la farine par analyse des gaz de l'espace de tête après 1 mois d'entreposage à 30°C selon J. Löliger, "65th Annual meeting of the potato association of America, University of Prince Edward Island, Charlottetown P.E.I. Canada, 2-6.08.1981". L'analyse de la teneur en pentane donne le degré d'oxydation étant donné que l'acide linoléique en s'oxydant donne lieu à la formation préférentielle de cet hydrocarbure. L'exemple de la stabilisation d'une farine de céréale constitue un bon modèle pour des aliments complexes séchés tels que ceux mentionnés ci-dessus.

Le tabeeau IV ci-dessous indique la quantité molaire de pentane ainsi que le pourcentage d'oxygène résiduel en volume des gaz de l'espace de tête.

**Tableau IV**

| Additif | $10^{-9}$ Mole Pentane | $O_2$-résiduel % en volume |
|---|---|---|
| Blanc | 2,344 | 19,59 |
| 500 ppm extrait de romarin du commerce | 0,077 | 20,80 |
| 1000 ppm acide tannique du commerce | 0,478 | 20,42 |
| 1000 ppm extrait de caroube selon l'exemple 1a | 0,106 | 20,61 |
| 1000 ppm Pentagalloylglucose (90 %) selon l'exemple 3 | 0,396 | 20,40 |

Légende:
Blanc: sans additif
ppm: partie par million

**Revendications**

1. Procédé de préservation des produits alimentaires et cosmétiques contre l'action de l'oxygène, caractérisé par le fait qu'on incorpore dans le produit à préserver une quantité efficace de gallotannins hydrolysables extraits à partir d'une matière végétale répondant à la formule

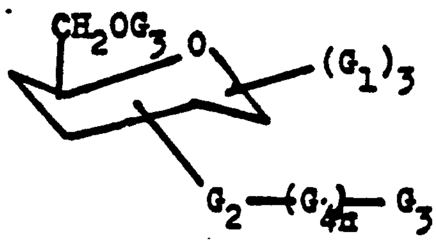

dans laquelle $G_1$ est un résidu d'acide gallique de formule

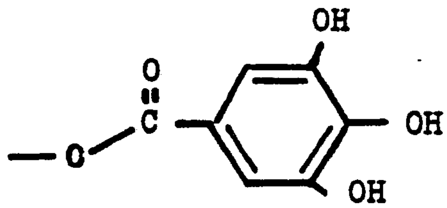

$G_2$ est un résidu d'acide gallique de formule

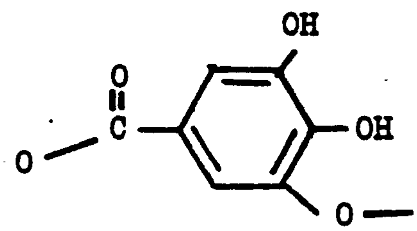

$G_3$ signifie

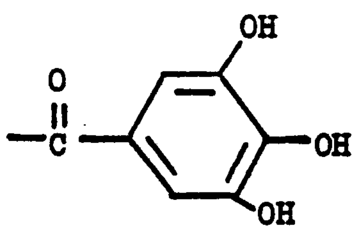

$G_4$ signifie

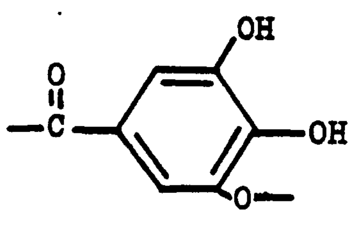

et n est 0,1 ou 2, le groupe $-G_2-[G_4]n-G_3$ étant en position 2, 3 ou 4, les groupes $G_1$ occupant les positions libres du noyau glucose.

2. Procédé selon la revendication 1, caractérisé par le fait que la matière végétale est constituée de gousses de caroube ou de résidus désucrés de celles-ci.

3. Procédé selon la revendication 1, caractérisé par le fait que les gallotannins sont présents sous forme d'extraits bruts obtenus par extraction de la matière végétale par un solvant miscible à l'eau et élimination du solvant.

4. Procédé selon la revendication 1, caractérisé par le fait que les gallotannins sont présents sous forme d'extrait purifié obtenu par extraction de la matière végétale par un solvant miscible à l'eau, élimination du solvant miscible à l'eau, mise en contact de l'extrait brut séché obtenu avec un mélange de solvant non miscible à l'eau et d'eau, séparation de la phase organique et élimination du solvant non miscible à l'eau.

5. Procédé selon la revendication 1, caractérisé par le fait que les gallotannins sont présents essentiellement sous forme de pentagalloylglucose obtenu par extraction de la matière végétale par un solvant miscible à l'eau, évaporation du solvant sous vide, mise en contact de l'extrait brut séché obtenu avec un mélange de solvant non miscible à l'eau et d'eau, séparation de la phase organique et évaporation du solvant sous vide, hydrolyse de l'extrait purifié et séché contenant essentiellement l'acide tannique dans un milieu solvant miscible à l'eau après avoir réglé le pH à 6-8 au moyen d'un milieu tampon, en l'absence d'oxygène, pendant 4 à 7 jours et évaporation du solvant.

6. Procédé selon la revendicaiton 5, caractérisé par le fait que l'extrait purifié, hydrolysé et séché est mis en suspension dans l'eau et dialysé contre l'eau pure de manière à en éliminer les petites molécules et les sels repris par un solvant organique, le solvant est évaporé et le résidu est séché.

7. Procédé selon l'une des revendications 1 à 6, caractérisé par le fait qu'on incorpore dans le produit à protéger 0,01 à 1 % et de préférence 0,05 à 0,2 % en poids de gallotannins hydrolysables.

**Patentansprüche**

1. Verfahren zum Schützen von Nahrungsmittel- und Kosmetikprodukten gegen eine Einwirkung von Sauerstoff, dadurch gekennzeichnet, daß man dem zu schützenden Produkt eine wirksame Menge von hydrolysierbaren Gallotanninen einverleibt, welche Gallotannine aus einem Pflanzenmaterial extrahiert worden sind und der Formel:

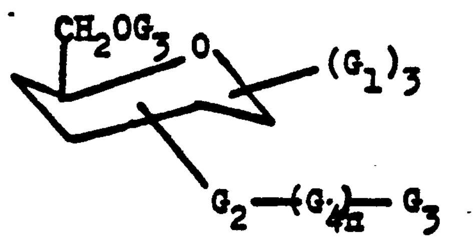

entsprechen, in welcher $G_1$ ein Gallussäurerest der Formel

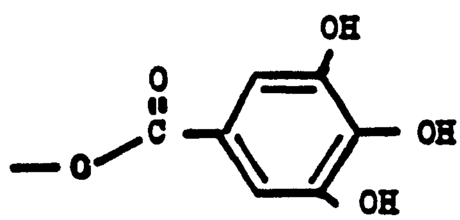

ist,

$G_2$ ein Gallussäurerest der Formel

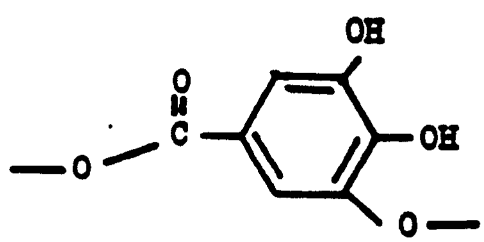

ist,

$G_3$

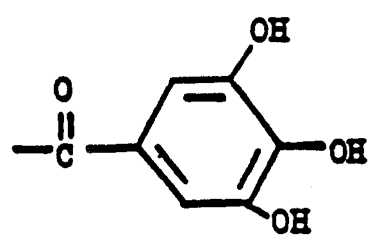

bedeutet, $G_4$

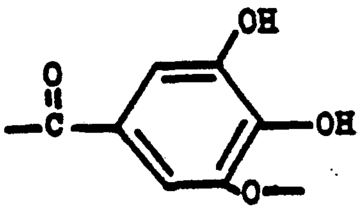

bedeutet und n 0, 1 oder 2 ist, wobei die Gruppe $-G_2-(G_4)_n-G_3$ in den Stellungen 2, 3 oder 4 vorliegt und wobei die Gruppen $G_1$ die freien Stellungen des Glucosekernes besetzen.

2. Verfahren nach Anspruch 1, dadurch gekennzeichnet, daß das Pflanzenmaterial aus Johannisbrotschoten oder aus entzuckerten Resten davon besteht.

3. Verfahren nach Anspruch 1, dadurch gekennzeichnet, daß die Gallotannine in der Form von Rohextrakten vorliegen, welche durch Extrahieren des Pflanzenmaterials mit einem mit Wasser mischbaren Lösungsmittel und Entfernen des Lösungsmittels erhalten worden sind.

4. Verfahren nach Anspruch 1, dadurch gekennzeichnet, daß die Gallotannine in der Form eines gereinigten Extraktes vorliegen, der durch Extrahieren des Pflanzenmaterials mit einem mit Wasser mischbaren Lösungsmittel, Entfernen des mit Wasser mischbaren Lösungsmittels, Inberührungbringen des so erhaltenen, getrockneten Rohextraktes mit einem Gemisch von mit Wasser nicht mischbarem Lösungsmittel und Wasser, Abtrennen der organischen Phase und Entfernen des mit Wasser nicht mischbaren Lösungsmittels erhalten worden ist.

5. Verfahren nach Anspruch 1, dadurch gekennzeichnet, daß die Gallotannine im wesentlichen in der Form von Pentagalloylglucose vorliegen, welche durch Extrahieren des Pflanzenmaterials mit einem mit Wasser mischbaren Lösungsmittel, Abdampfen des Lösungsmittels im Vakuum, Inberührungbringen des so erhaltenen, getrockneten Rohextraktes mit einem Gemisch von mit Wasser nicht mischbarem Lösungsmittel und Wasser, Abtrennen der organischen Phase und Abdampfen des Lösungsmittels im Vakuum, Hydrolyse des gereinigten und getrockneten Extraktes, der im wesentlichen die Gerbsäure enthält, in einem Medium aus mit Wasser mischbarem Lösungsmittel, nachdem der pH-Wert mittels eines Puffermediums auf einen Wert von 6 bis 8 eingestellt worden ist, in Abwesenheit von Sauerstoff, während 4 bis 7 Tagen, und Abdampfen des Lösungsmittels erhalten worden ist.

6. Verfahren nach Anspruch 5, dadurch gekennzeichret, daß der gereinigte, hydrolysierte und getrocknete Extrakt in Wasser in Suspension gebracht und gegen reines Wasser auf solche Weise dialysiert wird, daß die kleinen Moleküle und die Salze entfernt werden, daß der Extrakt anschliessend erneut mit einem organischen Lösungsmittel aufgenommen wird, das Lösungsmittel abgedampft wird und der Rückstand getrocknet wird.

7. Verfahren nach einem der Ansprüche 1 bis 6, dadurch gekennzeichnet, daß man dem zu schützenden Produkt 0,01 bis 1 Gew.-%, vorzugsweise 0,05 bis 02 Gew.-% hydrolysierbare Gallotannine einverleibt.

**Claims**

1. A method of protecting foodstuffs and cosmetic products against the effects of oxygen, characterized in that an effective quantity of hydrolyzable gallotannins extracted from plant matter and corresponding to the following formula is incorporated in the product to be protected:

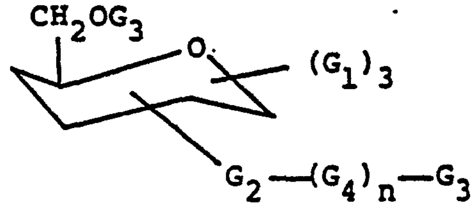

in this formula,

$G_1$ is a gallic acid residue corresponding to the following formula

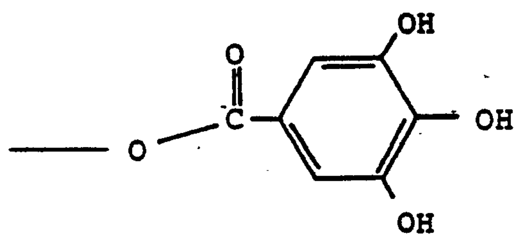

$G_2$ is a gallic acid residue corresponding to the following formula

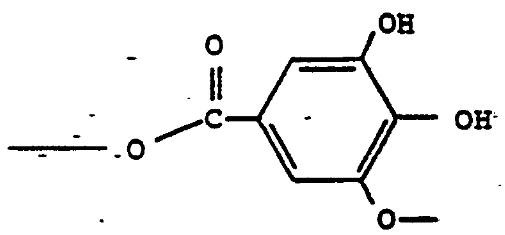

$G_3$ represents

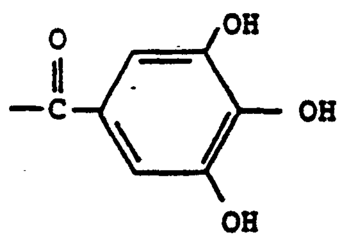

$G_4$ represents

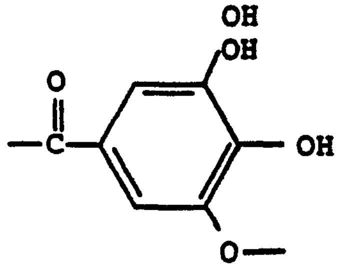

and n is 0, 1 or 2, the group $-G_2-[G_4]_n-G_3$ being in the 2, 3 or 4 position, the groups $G_1$ occupying the free positions of the glucose nucleus.

2. A method as claimed in Claim 1, characterized in that the plant matter consists of carob pods or desugared residues of carob pods.

3. A method as claimed in Claim 1, characterized in that the gallotannins are in the form of crude extracts obtained by extraction of the plant matter with a water-miscible solvent and elimination of the solvent.

4. A method as claimed in Claim 1, characterized in that the gallotannins are in the form of purified extract obtained by extraction of the plant matter with a water-miscible solvent, elimination of the water-miscible solvent, contacting of the dried crude extract obtained with a mixture of water-immiscible solvent and water, separation of the organic phase and elimination of the water-immiscible solvent.

5. A method as claimed in Claim 1, characterized in that the gallotannins are essentially in the form of pentagalloyl glucose obtained by extraction of the plant matter with a water-miscible solvent, evaporation of the solvent in vacuo, contacting of the dried crude extract obtained with a mixture of water-immiscible solvent

and water, separation of the organic phase and evaporation of the solvent in vacuo, hydrolysis of the dried and purified extract essentially containing tannic acid in a water-miscible medium after adjustment of the pH to 6-8 with a buffer medium in the absence of oxygen over a period of 4 to 7 days and evaporation of the solvent.

6. A method as claimed in Claim 5, characterized in that the dried, purified and hydrolyzed extract is suspended in water and dialyzed against pure water to eliminate the small molecules and the salts, taken up in an organic solvent, the solvent is evaporated and the residue is dried.

7. A method as claimed in any of Claims 1 to 6, from 0.01 to 1% and preferably from 0.05 to 0.2 % by weight of hydrolyzable gallotannins is incorporated in the product to be protected.